(19) Europäisches Patentamt
European Patent Office
Office européen des brevets

(11) Numéro de publication: **0 204 597 B1**

(12) # FASCICULE DE BREVET EUROPEEN

(45) Date de publication de fascicule du brevet: **08.01.92**

(51) Int. Cl.⁵: **A61K 31/505**

(21) Numéro de dépôt: **86401001.2**

(22) Date de dépôt: **12.05.86**

Le dossier contient des informations techniques présentées postérieurement au dépôt de la demande et ne figurant pas dans le présent fascicule.

(54) **Utilisation de l'alfuzosine pour le traitement des affections urinaires.**

(30) Priorité: **28.05.85 FR 8507950**

(43) Date de publication de la demande: **10.12.86 Bulletin 86/50**

(45) Mention de la délivrance du brevet: **08.01.92 Bulletin 92/02**

(84) Etats contractants désignés: **BE CH DE FR GB IT LI LU NL SE**

(56) Documents cités:

**BRITISH JOURNAL OF UROLOGY, vol. 48, 1976, pages 255-263; M. CAINE et al.: "The use of alpha-adrenergic blockers in Benign prostatic obstruction"**

**BRITISH JOURNAL OF PHARMACOLOGY, vol. 86, septembre 1985, page 416; I. CAVERO et al.: "alpha1-Adrenoceptor antagonist effects of alfuzosin in rabbit and dog lower urinary tract"**

(73) Titulaire: **SYNTHELABO**
**58, rue de la Glacière**
**F-75621 Paris Cedex 13(FR)**

(72) Inventeur: **Regnier, François**
**6, rue de la Source**
**F-54000 Nancy(FR)**

(74) Mandataire: **Thouret-Lemaitre, Elisabeth et al**
**SYNTHELABO Service Brevets 22, avenue Galilée**
**F-92352 LE PLESSIS ROBINSON CEDEX(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 204 597 B1

BRITISH JOURNAL OF UROLOGY, vol. 57, July 1985, pages 657-659; J.W.A. RAMSAY et al.: "A double-blind controlled trial of a new alpha-1 blocking drug in the treatment of bladder outflow obstruction"

BRITISH JOURNAL OF UROLOGY, vol. 47, 1976, pages 823-827; H.N. WHITFIELD et al.: "The effect of adrenergic blocking drugs on outflow resistance"

THERAPEUTIQUE NEUROLOGIE, vol. 36, no. 5, 21 janvier 1986, pages 223-228; M. PERRIGOT et al.: "Les alpha-bloquants dans les troubles vésico-sphinctériens"

BR. J. PHARMACOL, vol. 81, 1984, page 4; I. CAVERO et al.: "Alfuzosin (SL 77.499), a new antihypertensive agent with a peripheral site of action: II. In vitro pharmacological studies"

FED PROC., vol. 43, no. 3, 1984, page 734, abstract no. 2627; I. CAVERO et al.: "Alfulosine, an antipertensive agent with alpha1-adrenoceptor antagonist properties"

THE JOURNAL OF UROLOGY, vol. 133, février 1985, pages 298-303, The Williams & Wilkins Co., US; A. MATTIASSON et al.: "Adrenergic and non-adrenergic contraction of isolated urethral muscle from rabbit and man"

THE JOURNAL OF UROLOGY, vol. 130, août 1983, pages 275-280, The Williams & Wilkins Co., US; H. HEDLUND et al.: "Effects of prazosin in patients with benign prostatic obstruction"

## Description

La présente invention a pour objet des compositions pharmaceutiques contenant de l'alfuzosine.

L'alfuzosine de formule

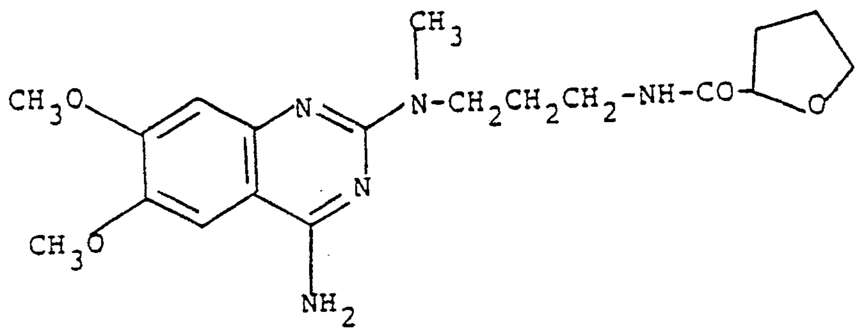

est connue pour son activité d'antihypertenseur. C'est un antagoniste des récepteurs $\alpha_1$-adrénergiques vasculaires qui possède des propriétés myorelaxantes directes (voir brevet français 78.03175 et C.A. 79.24373).

Chez beaucoup de malades présentant une dysurie on constate une hyperpression cervico-uréthrale que l'on rapporte à une hyperactivité relative des récepteurs $\alpha$-adrénergiques. (British Journal of Urology, 1976, 48, 255-263).

Les documents suivants

- British Journal of Pharmacology, vol 81, 1984, page 4
- FED. PROC. vol 43, n° 3, 1984, abstract 2627

traitent de l'activité antihypertensive et des propriétés antagonistes des récepteurs $\alpha_1$ de l'alfuzosine.

Le document J. Urol. 130, (1983) p. 275-280 a pour objet l'étude des effets de la prazosine chez des patients atteints d'une obstruction bénigne de la prostate.

La demanderesse a étudié l'activité de l'alfuzosine vis-à-vis des contractions induites par la phényléphrine sur des préparations de muscle lisse provenant de la base de la vessie (muscle trigone) et de l'urèthre de lapin.

Des lapins mâles (CEGAN) de 3 à 4 kg sont sacrifiés par exsanguination et dislocation cervicale.

La vessie et l'urèthre sont rapidement enlevées et placées dans une solution tiède de Krebs contenant du bicarbonate.

La composition de ce milieu de Krebs est la suivante en mM : NaCl 114 ; Kcl 4.7 - $CaCl_2$ 2.5 ; $KH_2PO_4$ 1.2 : $MgSO_4$ 1.2. ; $NaHCO_3$ 25.0 ; glucose 11.7 ; acide ascorbique 1.1. On ajoute du propranolol (1,0 $\mu$M) dans le milieu de Krebs pour bloquer les $\beta$-adrenocepteurs.

La vessie est ouverte transversalement et la région "trigone" du muscle, placée sur la surface dorsale de la vessie et entre les deux uretères, est disséquée.

On prépare également un anneau de 5 mm d'urèthre à partir de la région située entre la base de la vessie et la prostate.

Les parties de muscle trigone et d'urèthre sont lavées sous une tension de 1 g dans le milieu de Krebs.

On détermine la courbe-réponse de contraction aux concentrations cumulées de phényléphrine.

Les additions d'agoniste sont effectuées toutes les 5 mn. Les tissus sont lavés jusqu'à ce que la tension de base soit rétablie et sont ensuite incubés pendant 30 mn avec l'alfuzosine.

Une seconde courbe réponse à la phényléphrine est déterminée en présence d'alfuzosine.

Les courbes réponses aux concentrations de phényléphrine en présence ou absence d'alfuzosine sont exprimées en pourcentage de la réponse maximum obtenu par rapport à la courbe contrôle.

La puissance de l'alfuzosine est calculée sous la forme de $pA_2$ par la méthode de Schild, $pA_2$ = logarithme négatif de la concentration molaire d'alfuzosine qui provoque un déplacement vers la droite de la courbe réponse à l'agoniste.

L'alfuzosine (à une dose de 3.0 $\mu$M) provoque un déplacement parallèle vers la droite significatif de la courbe réponse à la phényléphrine à la fois dans le muscle trigone et dans l'urèthre.

L'alfuzosine provoque une réduction de 20 à 30 % des effets contractiles maximum de la phényléphrine.

Par analyse de Schild on peut déterminer le $pA_2$ qui est de 7.05 ± 0.17.

Des études cliniques ont permis également de montrer l'efficacité de l'alfuzosine chez les patients atteints d'une dysurie d'origine neurologique avec hypertonie uréthrale.

On injecte 5 mg d'alfuzosine par voie intraveineuse continue, pendant une durée de 20 mn. Les mesures sphinctérométriques, à l'aide d'un microcapteur électronique, ont été réalisées, avant et après l'injection du médicament, au niveau du col vésical et au niveau du sphincter strié de l'urèthre postérieur.

Les résultats de ces mesures ont permis de noter, au niveau du col vésical, une diminution de la pression de 44 % ($p < 0,001$), et, au niveau du sphincter strié, une diminution de 39 % ($p < 0,001$).

Une étude clinique a également été effectuée chez les paraplégiques.

Le paraplégique, homme spinal, réalise un modèle expérimental des récepteurs périphériques, car il est déconnecté de l'influence des centres nerveux supérieurs, diencéphaliques et corticaux.

L'hypertonie alpha-adrénergique est à l'origine de dysuries et de perturbations mictionnelles, étant donné la localisation des récepteurs alpha-adrénergiques dans l'urètre postérieur et le segment vésico-urétral ou col. L'ouverture du col et la baisse du gradient de pression dans l'urètre postérieur sont les deux conditions nécéssaires à la réalisation d'une miction efficace.

L'alfuzosine a été administrée par voie intraveineuse puis par voie orale si le premier test est positif.
Par voie intraveineuse, 5 mg d'alfuzosine sont injectés en 20 mn.

Après injection de l'alfuzosine, les pressions intraurétrales diminuent de façon significative.
Le test est considéré comme positif s'il y a déclenchement de la miction, c'est à dire obligatoirement ouverture du col.
Pour les patients pour lesquels le test est positif, l'administration de l'alfuzosine a été ensuite effectuée par voie orale à raison de 9 mg/24 h/28 j.

Dans la majorité des cas, le traitement per os a permis de rendre la miction plus facile à déclencher.

L'alfuzosine peut être utilisée pour le traitement des affections du bas appareil urinaire, dans lesquelles l'hyperactivité des récepteurs alpha-adrénergiques du système vésicosphinctérien perturbe le cycle continence/miction.

Il s'agit de patients tels que les hommes et les femmes qui présentent une maladie du col vésical, ou les hommes qui présentent une hypertrophie bénigne de la prostate avec dysurie d'origine alpha-adrénergique.

Il peut encore s'agir de patients atteints de troubles neurologiques, tels que la paraplégie ou la sclérose en plaque, pour lesquels la perturbation de la miction relève aussi de l'alfuzosine.

Les compositions pharmaceutiques de l'invention contenant l'alfuzosine en association avec tout excipient approprié peuvent être administrées par voie orale, parentérale ou transdermique. Elles sont présentées sous toute forme appropriée telle que gélule, comprimé, solution etc...
Les compositions pharmaceutiques peuvent également être présentées sous la forme de comprimés ou gélules retard.

La posologie quotidienne peut aller de 0,5 à 10 mg.

Des exemples de formulations pharmaceutiques sont donnés ci-dessous :

| Comprimé : | | mg |
|---|---|---|
| alfuzosine (sous forme de chlorhydrate) | | 5 |
| cellulose microscristalline | | 36 |
| lactose | | 122 |
| carboxymethylamidon sodique | | 7 |
| polyvidone excipient | | 9 |
| stéarate de magnésium | | 1 |
| | | 180 |
| enrobage | env. | 8 |

| Soluté injectable | | mg |
|---|---|---|
| alfuzosine (sous forme de chlorhydrate) | | 1 |
| chlorure de sodium | | 44.9 |
| eau pour préparations injectable | qsp | 5 ml |

**Revendications**

1. Utilisation de l'alfuzosine pour la fabrication de médicaments destinés au traitement des affections urinaires.

**Claims**

1. Use of alfuzosine for the manufacture of drugs intended for the treatment of urinary diseases.

**Patentansprüche**

1. Verwendung von Alfuzosine zur Herstellung von Arzneimitteln zur Behandlung von Harnkrankheiten.